(19) Europäisches Patentamt
European Patent Office
Office européen des brevets

(11) Publication number: **0 055 092**
**B1**

(12) **EUROPEAN PATENT SPECIFICATION**

(45) Date of publication of patent specification: **17.04.85**

(51) Int. Cl.⁴: **C 08 G 12/30,** C 07 D 251/30, C 08 L 61/26, C 08 K 5/34

(21) Application number: **81305946.6**

(22) Date of filing: **18.12.81**

# (54) Acetals of adducts of acrolein and isocyanuric acid and polymer compositions thereof.

(30) Priority: **22.12.80 US 218900**
**22.12.80 US 219208**

(43) Date of publication of application:
**30.06.82 Bulletin 82/26**

(45) Publication of the grant of the patent:
**17.04.85 Bulletin 85/16**

(84) Designated Contracting States:
**BE DE FR GB IT**

(56) References cited:
**DE-C- 918 780**
**FR-A-1 339 408**
**GB-A- 542 974**
**US-A-3 737 432**

(73) Proprietor: **MONSANTO COMPANY**
**Patent Department 800 North Lindbergh Boulevard**
**St. Louis, Missouri 63166 (US)**

(72) Inventor: **Cohen, Saul Mark**
**15 Lindsay Road**
**Springfield Massachusetts 01128 (US)**
Inventor: **LeBlanc, John Roger**
**34 Decorie Drive**
**Wilbraham Massachusetts 01095 (US)**

(74) Representative: **Pearson, John Lionel et al**
**Monsanto Europe S.A. Avenue de Tervuren**
**270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 055 092 B1

Courier Press, Leamington Spa, England.

**Description**

This invention relates to acetals prepared by reaction of monohydric alcohols and adducts of acrolein and isocyanuric acid and to a process of preparation of such acetals. In particular, this invention relates to acetal compositions comprising acetals of (3-oxopropyl)isocyanurates and to a process of preparation of such acetals. It also relates to compositions comprising such acetals and polyfunctional acetal-reactive compounds, to the polymeric reaction products of such compositions and to articles comprising such reaction products.

The process of the invention for the preparation of an acetal comprises:

a. slurrying isocyanuric acid in a solvent medium of pH in the range of about 5 to about 7, containing at least about 1 mole of acrolein per mole of isocyanuric acid;

b. allowing the acrolein to add to the isocyanuric acid at a temperature in the range of about 50 to about 120°C; and

c. allowing the adduct to react with a monohydric primary or secondary alcohol in the presence of a strong acid under conditions which cause the water released by the reaction to be evolved from the reaction medium.

The acetals contain various degrees of 3,3 - (dihydrocarbyloxy)propyl substitution at the isocyanurate nitrogen atoms depending on the combined acrolein content of the reacted adduct and the amount of monohydric alcohol used with it. Preferably the adducts are formed by the addition of from 2 to 3 moles of acrolein per mole of isocyanuric acid. The acetals form stable solutions of low viscosity.

The solvent medium in step a. of the process of the invention contains at least about 1 mole of acrolein per mole of isocyanuric acid, and preferably in step b. at least about one mole of acrolein reacts with one mole of isocyanuric acid to form an aldehyde containing an average of about one 3-oxopropyl group per isocyanurate ring. More preferably at least about 2 moles of acrolein react with one mole of isocyanuric acid to form an aldehyde containing an average of about two 3-oxopropyl groups per isocyanurate ring, and most preferably about three moles of acrolein react per mole of isocyanuric acid to provide an aldehyde, containing tris(3-oxopropyl)isocyanurate as the major fraction, (hereinafter referred to as isocyanuric trialdehyde).

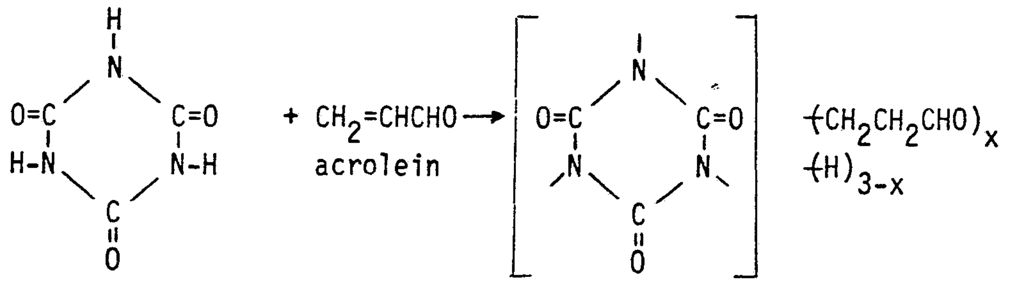

isocyanuric acid (3-oxopropyl)isocyanurates (x≤3)

Under normal conditions, isocyanuric acid is the predominant tautomer in "cyanuric acid", comprising about 94 mole percent of the tautomeric mixture. In the reaction with acrolein, the isocyanuric acid tautomer reacts more readily than the cyanuric acid tautomer so that the reaction product may contain more than 94 percent of the isocyanurate derivative. However, to the extent that cyanurates are formed, since they contain an equal number of aldehyde groups, they contribute equally to the aldehyde functionality of the product aldehyde. For the purposes of this disclosure, it is understood that the term "isocyanuric acid" includes isocyanuric acid containing a minor amount of cyanuric acid tautomer.

Several commercial grades of "cyanuric acid" are available, containing various amounts of impurities such as ammelide and ammeline and ammelide- and ammeline-sulfamic acids. While any of these grades are suitable for the addition of acrolein, it is preferred that the "cyanuric acid" contain at least about 98 weight percent of the isocyanuric acid and cyanuric acid tautomers, and ammelide- and ammeline-sulfamic acids in the range of about 0.1 to about 0.3 weight percent.

The reaction temperature in step b. of the process is preferably in the range of about 80 to about 100°C., and more preferably in the range from about 88 to about 92°C to achieve a fast rate of reaction without excessive formation of insoluble polymer.

The solvent medium in the process can be acrolein in substantial excess over the stoichiometric amount for reaction with isocyanuric acid, or it can be selected from solvents for the aldehyde reaction product which are inert to aldehydes under the conditions of the reaction, and can optionally contain an excess of acrolein over the amount desired to be added to the isocyanuric acid. Excess acrolein can be used, especially when a high degree of acrolein addition and a fast reaction rate are desired. Thus, depending upon the desired degree of acrolein addition, the amount of acrolein can range from about 1 mole to about 6 moles or more per mole of isocyanuric acid. In general the range of solvents for the isocyanuric monoaldehyde is rather limited and includes solvents such as water, dimethylformamide, dimethylacetamide and methyl sulfoxide which are inert but highly polar. In contrast the range of solvents

for the isocyanuric trialdehyde is quite broad and includes ethers, esters, ketones and hydrocarbons or mixtures thereof. Among the preferred solvents for the trialdehyde are the lower boiling ethers, esters and ketones since they can be readily evaporated when the reaction is complete.

The pH of the solvent mediuum in step a. of the process is in the range of about 5 to about 7. pH is measured by applying a few drops of reaction solution to universal pH paper or some comparable pH color indicator, allowing the solvent to evaporate and adding a few drops of methanol to the pH paper. When the methanol has evaporated, the color of the pH paper is compared with the color standard and the pH value thus obtained is considered to be the pH of the reaction solution.

Preferably the pH is maintained in the range of about 5 to about 7 by addition of a salt of an amine of $pK_b$ in the range of about 3.5 to about 9.5 and an acid of $pK_a$ in the range of about 1 to about 5.

As will be evident from well-established principles, the quantity of acid required for control of the acidity within the specified limits will be at least that required to react with all the amine plus an amount in excess thereof sufficient to keep the apparent pH of the reaction mixture below 7.0. As will also be evident from well-established principles, the amount of acid to be employed over and above that required for reaction with the amine will vary from acid to acid and amine to amine, dependent upon the dissociation constants of the compounds involved. Thus, in the case of a very weak amine and a strong acid, a slight amount of the acid over the stoichiometric equivalent would be sufficient. Conversely, with a strong amine base and weak acid, stoichiometric equivalents of acid to amine as high as 3 to 1 may be indicated. In the case of the amphoteric amine compounds, the use of acid can be dispensed with, but it is not necessary to do so provided the quantity used is insufficient to take the pH of the reaction outside the specified range. In some instances the use of a small quantity of acid with the amphoteric amine compound also may be found advantageous.

The amine can be selected from primary, secondary and tertiary amines, polyamines, and the like, including amphoteric amine compounds. These amines can be aliphatic or aromatic or mixed aliphatic and aromatic, including carbocylic and heterocyclic. By the term "amphoteric amine compound", as used herein, is meant an amine of the classes listed above which also contains in the molecule an acidic hydrogen atom connected to a carbon atom through an oxygen atom, as in the case of the aminoacids and aminophenols by way of illustration. Representative of the useful amines are the following: monomethylamine, dimethylamine, trimethylamine, monoethylamine, diethylamine, triethylamine, monopropylamine, dipropylamine, tripropylamine, monobuutylamine, dibutylamine, tributylamine, and mono-, di-, and tri-alkyl amines containing up to seven carbon atoms to the alkyl group; aniline, diphenylamine, toluidine, monomethylaniline, dimethylaniline and other arylamines and alkyl-substituted arylamines containing up to seven carbon atoms to the group attached to the nitrogen atom; monoethanolamine, diethanolamine, triethanolamine, diethylene triamine, triethylene tetramine, phenylenediamines, and other aliphatic and aromatic polyamines containing not more than seven carbon atoms to the aliphatic or aromatic group present therein. Representative of the amphoteric amine compounds that can be used as catalyst in our process are glycine, beta-alanine, anthranilic acid and aminophenols.

The acid can be selected from a wide variety of acids including formic, acetic, propionic, butyric, valeric, hexanoic, heptanoic, octanoic, oxalic, succinic, glycolic, lactic, benzoic, phthalic and sulfamic.

In general, the preferred amines are tertiary amines such as trimethylamine, triethylamine and tripropylamine and the preferred acids are the lower carboxylic acids such as formic acid, glycolic acid and lactic acid.

The amine salt concentration is not narrowly critical but may be varied within moderate limits. A concentration in the range of about 0.1 to about 1.0 mole per hundred moles of isocyanuric acid is preferred. Within this range the exact amount employed depends to some extent on the particular amine salt selected and the reaction temperature. At concentrations below about 0.1 mole of amine salt per 100 moles of isocyanuric acid, an undesirable formation of acrolein oligomers competes with the formation of the desired adduct of isocyanuric acid, and crosslinked product also forms.

When the reaction has been carried to completion, the amine salt can be removed by treatment of the reaction solution with a suitable adsorbent or absorbent for the amine salt such as a diatomaceous earth, finely divided silica, alumina, animal charcoal, an ion-exchange resin or a combination of ion-exchange resins. The treatment can be carried out in any convenient way, such as by stirring the solution with the sorbent for sufficient time to substantially sorb the amine salt and filtering off the sorbent, or by passing the reaction solution through a bed of sorbent. After removal of the amine salt, sufficient acid of moderate strength with pKa in the range of about 1 to about 3 may be added to produce a final product with a pH in the range of about 1 to about 3 and more preferably in the range of about 1.5 to 2. Suitable acids include oxalic acid, dichloroacetic acid, glycerophosphoric acid, maleic acid, 2 - methyl - 6 - nitrobenzoic acid and phosphorous acid. The solution of the aldehyde reaction product is then stripped to remove solvent. When the reaction solvent is provided by an excess of acrolein, removal of the excess acrolein is preferably achieved by a multicycle addition of inert solvent and removal of the solvent by vacuum stripping and finally water is added and removed by vacuum stripping. Suitable solvents for the stripping process are ethers, esters and ketones of boiling point in the range of about 60 to about 100°C.

Any commercial grade of acrolein is suitable for the reaction with isocyanuric acid. Such grades generally contain up to about 4 weight percent water. Also from about 0.1 to about 0.25 weight percent

hydroquinone can be present as a polymerization inhibitor. While water up to about 4 percent is tolerable and has little effect on the acroleinisocyanuric acid reaction, we have found that when the water content is reduced to less than about 1.0 percent, the amount of polyacrolein produced during the reaction is decreased. The tendency of acrolein to dimerize by a Diels-Alder addition has long been recognized. Acrolein containing from about 0.1 to about 2.0 percent of dimer yields greater than the theoretical amount of the desired aldehyde upon reaction of the acrolein with isocyanuric acid. However, analysis by thin layer chromatography reveals no difference in the aldehyde prepared from such acrolein compared with aldehyde prepared by reaction of isocyanuric acid and acrolein containing less than 0.1 percent dimer.

In step c. of the process of this invention, the aldehyde adduct is reacted with a monohydric primary or secondary alcohol. The reaction is preferably carried out in an inert solvent medium which is a solvent for the acetal and at least one of the reactants. More preferably, a solvent is selected which allows the water of reaction to be removed by azeotropic distillation. Such solvents include aliphatic and aromatic hydrocarbon solvents such as hexane, heptane, octane, benzene, toluene and xylene, higher boiling alcohols such as butyl alcohol, amyl alcohol, hexyl alcohol and benzyl alcohol and chlorinated hydrocarbons such as chloroform, carbon tetrachloride and ethylene dichloride. Acid catalysts which may be used are preferably strong acids of $pk_a$ less than 3 such as the mineral acids, oxalic acid and alkyl-, fluoroalkyl- and aryl-sulfonic acids and fluorophosphoric acids, including methanesulfonic acid, trifluoromethanesulfonic acid, benzenesulfonic acid, toluenesulfonic acid and hexafluorophosphoric acid. While the amount of acid catalyst is not critical it is preferably selected in the range of about 0.05 to about 1 weight percent of the aldehyde. The temperature of reaction is not critical and is generally determined by the boiling point of the reaction medium. However higher or lower temperatures can be used by carrying out the reaction in a closed vessel to provide super- or sub-atmospheric conditions. The course of the reaction can be followed by decrease of the aldehyde carbonyl band in the infra-red spectrum of the isocyanuric aldehyde. When the reaction is complete, the acid catalyst is removed by conventional means for example by neutralization with an inorganic base and filtration of the salt which is formed or by neutralization with an ion exchange resin. Preferred inorganic bases form insoluble salts in the reaction medium and include the oxides and hydroxides of group II A metals, such as magnesium and calcium oxides and hydroxides. While any primary or secondary monohydric alcohol can be used to prepare the acetals of the present invention, when the acetal is to be used as a polymerization or cross-linking agent for polyfunctional compounds containing acetal-reactive groups such as polyhydroxy compounds, it is preferred to use $C_1$ to $C_8$ alcohols since they can be more readily displaced during the polymerization or cross-linking reaction. Among the preferred alcohols are methyl, ethyl, propyl, isopropyl, butyl, cyclohexyl and benzyl alcohols. The higher boiling alcohols can be used advantageously both as the acetalizing agent and as the azeotropic solvent. More preferably, when the acetal is to be used as a polymerization or cross-linking agent it is preferred that it be the acetal of methanol. The products of acetalization of the aldehydes with primary or secondary monohydric alcohols are [3,3 - di(hydrocarbyloxy)propyl]isocyanurates. Thus acetalization of the trialdehyde with methanol yields an acetal comprising substantially tris(3,3 - dimethoxypropyl)isocyanurate. Complete acetalization can be achieved by effecting reaction of about 2 moles of monohydric alcohol per aldehyde group. The assumption is made that any oligomeric or associated aldehyde reverts to the monomeric or unassociated state, and is consumed in the reaction. Excess of the monohydric alcohol allows the reaction to be completed more rapidly. When less than the stoichiometric quantity of monohydric alcohol for reaction with the aldehyde groups is used in the acetalization reaction, partial acetalization occurs to provide products which nevertheless are more soluble than the aldehydes and hence more amenable to reaction with aldehyde-reactive and acetal-reactive compounds. Preferably at least about 50 percent of the aldehyde groups of the isocyanuric aldehyde are acetalized.

In the compositions of the invention comprising a polyfunctional acetal-reactive compound and an acetal, the acetal-reactive compounds may contain reactive groups such as primary or secondary amines, primary and secondary amides, alcohols, thiols, phenols, anilines and oxiranes.

Crosslinking or polymerization reactions are readily achieved with the acetals by reacting them with polyols under strong acid conditions to produce polyacetals. The acetals of the monoaldehyde are effective but the di- and tri-aldehydes are preferred since they can provide more efficient crosslinking. The temperature of the reaction is not critical and is selected, preferably in the range of about 80 to about 150°C to provide the appropriate degree of reaction in a convenient time. Suitable acid catalysts include those listed above for the acetalization of the aldehydes by monohydric alcohols.

The reaction is carried out in a solvent inert to the reactants. When the polyols are of molecular weight of about 10,000 or less, high solids coating systems can be obtained containing at least about 50 percent solids and more preferably at least about 70 solids.

Suitable polyols for transacetalization reaction include oligomeric and polymeric hydroxy compounds containing a plurality of hydroxy groups per molecule, such as hydroxy-containing polyester and alkyds, polyethers such as polyethylene glycols, polypropylene glycols and polytetramethylene glycols, polyvinyl alcohol and copolymers of vinyl alcohol, polymers of allyl alcohol, polymers of hydroxyacrylates such as 2-hydroxyethyl acrylate and 2-hydroxyethyl methacrylate, starches, starch derivatives, celluloses and cellulose derivatives. Also suitable are the simple polyols such as 1,4-butanediol, 1,6-hexanediol, 1,4-di-(hydroxymethyl)cyclohexane, cyclohexanediols, trimethylolethane, trimethylolpropane, glycerol,

pentaerythritol, sorbitol, glucose and sucrose. Diols with hydroxy groups in 1,2- or 1,3-placement, such as ethylene glycol and 1,3-propanediol, which form cyclic acetals may be used as monofunctional reactants for control of the molecular weight of the polyacetals.

Suitable polythiols for reaction with the acetals of the present invention include low molecular weight di- and tri-thiols such as 1,2-ethanedithiol, 1,4-butanedithiol, and 1,6-hexanedithiol, and high molecular weight polythiols such as poly(ethylene thiol), poly(p-styrenethiol) and poly(4-mercaptomethylstyrene).

Among the polyamines which can be reacted with the acetals of the present invention are low molecular weight hydrocarbyldiamines and polyamines and poly(hydrocarbylpolyamines) such as 1,2-ethanediamine, 1,6-hexanediamine and 1,12-dodecanediamine, diethylene triamine and tetraethylene pentamine, and intermediate and high molecular weight poly(hydrocarbylpolyamines), poly(alkylenimines) and vinylamine polymers.

Polyamides suitable for the reaction with the acetals of the present invention include urea, guanidine, the polyamides of polycarboxylic acids such as adipamide and dimer acid diamide, and high molecular weight polyamides such as the nylons, polypeptides and polyacrylamide.

Polyoxiranes which can be used for reaction with the acetals of the present invention include those set forth in the Encyclopaedia of Polymer Science and Technology, Wiley 1967, Vol. 6, pp. 212—219 and include the diglycidyl ether of bisphenol-A and oligomers thereof, expoxidized novolacs, aliphatic polyglycidyl ethers, and peracetic epoxidized products such as vinyl cyclohexene dioxide and epoxidized polybutadiene.

Polymerizable compositions containing the acetals and the polyfunctional compounds are obtained by blending the acetals and the polyfunctional compounds in the mole ratio from about 1:m/2 to about n/2:1, where m is the total of acetal, hemiacetal and aldehyde groups per isocyanurate ring of the acetal composition, the aldehyde groups being assumed to be available in the monomeric or unassociated form, and n is the functionality of the polyfunctional compound, both m and n being about 2 or more to achieve chain extension.

The acetal groups can act as monofunctional or difunctional groups depending upon the type and placement of the acetal-reactive groups with which the acetal is reacted. Thus the acetal group will be monofunctional when it is reacted with a polyol with hydroxy groups in 1,2- or 1,3 placement to one another to form a cyclic acetal. On the other hand, the acetal group will be difunctional when it reacts with hydroxy groups which are not in such 1,2- or 1,3-placement to one another.

The acetals in which substantially complete acetalization has been achieved with monohydric alcohols, provide a convenient stable source of essentially monomeric (3-oxopropyl)isocyanurates for reactions in aqueous media, since they are readily hydrolyzed when they are added to aqueous media containing a catalytic amount of acid.

The polymerizable compositions comprising polyfunctional compounds and the acetals of the present invention can be used in a multiplicity of applications. They can be used as adhesives and laminating agents, bonding resins, surface coating systems and molding resins. They can be modified by the addition of pigments, plasticizers, colorants, dyes, pigment dispersing agents, flow control agents or stabilizers.

The following Example illustrates the invention. Unless otherwise indicated, all parts and percentages are by weight.

Example

(a) Preparation of isocyanuric trialdehyde

To a pressure kettle (rated for 3.45 MPa (500 psi)), 526 parts by weight of freshly distilled acrolein containing 100 parts per million 2,6 - di - t - butyl - 4 - methylphenol, 200 parts per million hydroquinone and 100 parts per million formic acid is charged. To the stirred acrolein is gradually added 646 parts by weight of 99.2% isocyanuric acid, to form a slurry. Another 500 parts by weight of acrolein is added to the stirred slurry, followed by 26 parts by weight of acrolein containing 2.4 parts by weight of triethylamine and 1.1 parts by weight of 90% formic acid dissolved therein. The isocyanuric acid contains 0.2 weight percent ammelide- and ammeline-sulfamic acids.

The kettle is sealed, and the stirred reactants are heated gradually to 90°C over a 25-minute period. An exotherm occurs at about 90°C; the initial reaction pressure is 170—210 kPa. Reaction temperature is controlled between 89—91°C by steady cooling through the cooling coils of the kettle. Controlled reaction is maintained at 89—91°C for a total of 45 minutes. However, after 35—40 minutes of reaction, a noticeable decrease in the exothermic rate is observed via the decreased amount of cooling required. The final reaction pressure is about 35 kPa.

After 45 minutes at 90°C, the reaction is terminated by cooling rapidly. Simultaneously 1600 parts of methyl ethyl ketone containing 5 parts of 90% formic acid is pumped into the stirred contents and stirring is continued with cooling until the temperature is ~20°C.

The reaction solution is drained from the kettle. It is filtered once through a Celite bed on a Buchner funnel; and twice through filter paper on a Buchner funnel. To the clear yellow filtrate then is added 15.0 parts of oxalic acid and the solution is stripped in a rotating evaporator at a water bath temperature of 60°C and an absolute pressure of 120 to 200 mm Hg. When the solvent has been removed by distillation, 800 parts of fresh methyl ethyl ketone is added to dissolve the product. The same stripping procedure is followed. At the end of the second stripping operation, the bath temperature is raised rapidly to 100°C and

maintained at that temperature for about 5 minutes. The residual product, a turbid viscous liquid of 3500 Pas viscosity at 80°C, then is mixed with 1500 parts of water and the solution is stripped at 105°C/180—18 mm/30 min to produce the product. This washing procedure is repeated twice more. The final product is poured into an aluminum tray. When allowed to cool to room temperature, it rapidly hardens to a glassy solid which crystallizes or becomes opaque after 1—48 hours. 1506 parts by weight of product are obtained corresponding to a yield of 100%. The opaque white, glassy solid can be ground up to a white powder. The ratio of aldehyde carbonyl infrared absorbance at 1740 $cm^{-1}$ to isocyanurate absorbance at 780 $cm^{-1}$ is 1.8. The aldehyde content determined by the quantitative hydroxylamine method corresponds to an equivalent weight of 101.6. The residual acrolein monomer, determined by gas chromatography is less than 100 parts per million of product.

When the reaction product is subjected to thin layer chromatography by the general procedure described in Egon Stahl's "Thin Layer Chromatography", Springer-Verlay, Heidelberg-New York, 2nd Ed. 1969 (English Translation) on EM Silica Gel-60-F-254 with ethyl acetate elution (2×) and visualizations by means of UV light (254 nm) for double bonds; dichlorofluorescein; AHTT aldehyde detection agent; and a solution of chlorine in o-toluidine for NH and $NH_2$ detection, four aldehyde components, 1 major corresponding to tris (3-oxopropyl)isocyanurate and 3 lesser, are separated. Two of the lesser ones may be polymeric. A trace of a fifth component, possibly acrolein oligomer, is also observed.

The method of Example 1 is repeated several times and the reaction products are subjected to analysis by a gel permeation chromatography technique. This method employs two Varian Micropak® TSK columns (sizes 1000H and 2000H in sequence, each 50 cm in length) which can separate materials by size from 100 to about 10,000 molecular weight. Coupled with a UV spectrophotometer detector set at 220 nm wavelength, a tetrahydrofuran solution of the products is separated into 13 components. These consist of 3 groups: polymeric species, isocyanuric acid—acrolein adducts, and acrolein oligomers. Peak height ratios are used to determine relative percentages of the components. The following range is obtained:

| | |
|---|---|
| monomeric isocyanuric-acrolein adducts | 39—62% |
| polymer component | 39—58% |
| acrolein oligomers | 0.3—3.9% |

The range of tris-, bis- and mono-(3-oxopropyl) isocyanurates in the monomeric component are determined to be:

| | |
|---|---|
| tris-(3-oxopropyl)isocyanurate | 39—71% |
| bis-(3-oxopropyl)isocyanurate | 21—49% |
| mono-(3-oxopropyl)isocyanurate | 4—14% |

The precision of the analysis for the monomeric and polymeric components is ±4%.

$^{13}C$ NMR analysis of the reaction product displays the following peaks:

| Peak (ppm) | Assignment |
|---|---|
| 200 | saturated aldehyde carbonyl |
| 148 | isocyanuric acid carbonyl |
| 40.4 | α-methylene of saturated aldehyde |
| 35.8 | methylene attached to ring nitrogen |

Quantitatively, $^{13}C$ NMR has measured ranges of 2.0 to 2.7 aldehydes per isocyanuric acid ring.

(b) Preparation of a tris(3,3-dimethoxypropyl)isocyanurate composition

To a kettle equipped with stirrer, thermometer, reflux condenser and holding tank, there is charged 143 parts by weight of the adduct of acrolein and isocyanuric acid of part (a) and 46 parts by weight of methanol. The mixture is warmed until a clear solution of the hemiacetal forms. An additional 142 parts methanol and 125 parts of toluene is charged and is followed by 187 parts methanol containing 3 parts p-toluenesulfonic acid monohydrate. The reaction solution is refluxed for one hour, and 400 parts of solvent containing 14.4 parts of water is then removed by distillation. A mixture of 375 parts of methanol and 125 parts of toluene is added again, and the reaction solution is refluxed for 20 minutes and distilled to remove another 8.8 parts of water. The preceding step is again repeated, and a final 2.9 parts of water is

removed. Total water of reaction is 100% of theoretical. The reaction solution is cooled to room temperature and 2.36 parts of calcium hydroxide is added. After 20 minutes of stirring, the slurry is filtered, and the filtrate is stripped under reduced pressure to remove the solvent. The oily residue is dissolved in dry toluene, filtered and again stripped under reduced pressure. 207 g of crude tris(3,3-dimethoxypropyl)isocyanurate, corresponding to 99% yield, is obtained as a viscous, clear, dark red liquid which solidifies to a tan waxy solid, mp 45—52°C. The aldehyde content is 2.1 percent of the initial aldehyde content calculated for 100 percent tris(3-oxopropyl)isocyanurate starting material. The infrared spectrum of the product has a strong acetal peak at 1140 $cm^{-1}$.

When the crude tris(3,3-dimethoxypropyl)isocyanurate is suspended in an equal weight of water containing 5% p-toluenesulfonic acid and the slurry is heated at 60°C for 10 minutes, a clear solution is obtained and the crude tris(3,3-dimethoxypropyl)isocyanurate is converted back to crude tris(3-oxopropyl)-isocyanurate. The infrared spectrum now includes an aldehyde carbonyl bond approximately equal in intensity to the aldehyde carbonyl bond of the initial tris(3-oxopropyl)isocyanurate mixture.

**Claims**

1. A process for the preparation of an acetal which comprises:

a. slurrying isocyanuric acid in a solvent medium of pH in the range of about 5 to about 7 containing at least about 1 mole of acrolein per mole of isocyanuric acid;

b. allowing the acrolein to add to the isocyanuric acid at a temperature in the range of about 50 to about 120°C; and

c. allowing the adduct to react with a monohydric primary or secondary alcohol in the presence of a strong acid under conditions which cause the water released by the reaction to be evolved from the reaction medium.

2. The process of Claim 1 wherein the amount of acrolein at step (a) is in the range of about 1 to about 6 moles per mole of isocyanuric acid, wherein excess acrolein is removed at the end of step (b) and wherein the amount of monohydric alcohol reacted at step (c) is in the range of about 1 to about 2 moles per mole of combined acrolein.

3. The process of Claim 1 wherein the isocyanuric acid-acrolein reaction at step (b) is carried out at a temperature in the range of about 80 to about 100°C and wherein about 2 moles of monohydric alcohol per mole of combined acrolein is reacted at step (c).

4. The process of Claim 1 wherein the mildly acid condition at step (a) is obtained by addition of from about 0.1 to about 1.0 mole of an amine salt per 100 moles of isocyanuric acid and wherein the acid of step (c) has a $pk_a$ of less than 3 and is present in the range of about 0.05 to about 1 weight percent of the acrolein-isocyanuric acid adduct.

5. The process of Claim 4 wherein the amine salt is triethylamine formate.

6. The process of Claim 4 wherein the acid catalyst is neutralized with an inorganic base or an ion-exchange resin upon completion of step (c).

7. The process of Claim 1 wherein the monohydric alcohol is a $C_1$ to $C_8$ monohydric alcohol.

8. The process of Claim 1 wherein the monohydric alcohol is methyl alcohol.

9. An acetal of an isocyanuric acid-acrolein adduct and a monohydric primary or secondary alcohol obtainable by the process of Claim 1.

10. The acetal of Claim 9 wherein the monohydric alcohol is a $C_1$—$C_8$ alcohol.

11. The acetal of Claim 9 wherein the monohydric alcohol is methyl alcohol.

12. The acetal of Claim 9, 10 or 11 wherein the adduct comprises from 1 to 3 moles combined acrolein per mole of isocyanuric acid.

13. The acetal of Claim 10, 11 or 12 wherein the adduct comprises from 2 to 3 moles combined acrolein per mole of isocyanuric acid.

14. An acetal composition comprising a [3,3 - di - (hydrocarbyloxy)propyl]isocyanurate.

15. An acetal comosition comprising tris [3,3 - di - (hydrocarbyloxy)propyl]isocyanurate.

16. The acetal composition of Claim 14 or 15 wherein the hydrocarbyl group contains 1 to 8 carbon atoms.

17. The acetal composition of Claim 14 or 15 wherein the hydrocarbyl group is methyl.

18. A composition comprising a polyfunctional acetal-reactive compound and an acetal of the acroleinisocyanuric acid adduct and a monohydric primary or secondary alcohol obtainable by the process of Claim 1.

19. The composition of Claim 18 wherein the adduct comprises from about 1 to about 3 moles of acrolein added per mole of isocyanuric acid and is acetalized with from about 1 to about 2 moles of monohydric alcohol per mole of combined acrolein.

20. The composition of Claim 18 wherein the monohydric alcohol is a $C_1$ to $C_8$ alcohol.

21. The composition of Claim 18 wherein the monohydric alcohol is methyl alcohol.

22. The composition of Claim 18, 19, 20 or 21 wherein the mole ratio of acetal to polyfunctional acetal-reactive compound is in the range of about 1:m/2 to about n/2:1 where m is the functionality of the acetal and n is the functionality of the polyfunctional acetal-reactive compound and m and n are at least about 2.

23. The composition of Claim 22 wherein the functional groups of the acetal-reactive compound are selected from the group consisting of alcohol, thiol, oxirane and amide.

24. The composition of Claim 22 wherein the aldehyde-reactive compound is a polyhydroxy compound.

25. The reaction product of the composition of Claim 22, 23 or 24.

26. An article comprising the reaction product of the composition of Claim 22, 23 or 24.

**Patentansprüche**

1. Verfahren zur Herstellung eines Acetals, wobei

a. Isocyanursäure in einem Lösungsmedium mit einem pH-Wert im Bereich von etwa 5 bis etwa 7, welches mindestens etwa 1 Mol Acrolein pro Mol Isocyanursäure enthält, aufgeschlämmt wird,

b. Acrolein sich bei einer Temperatur im Bereich von etwa 50 bis etwa 120°C an die Isocyanursäure anlagert, und

c. das Addukt mit einem einwertigen primären oder sekundäre Alkohol mit einer Hydroxylgruppe in Gegenwart einer starken Säure unter Bedingungen, die bewirken, daß das durch die Reaktion freigesetzte Wasser aus dem Reaktionsmedium ausgeschieden wird, umgesetzt wird.

2. Verfahren nach Anspruch 1, worin die Menge an Acrolein in Stufe (a) im Bereich von etwa 1 bis etwa 6 Mol pr Mol Isocyanursäure liegt, worin überschüssiges Acrolein am Ende der Stufe (b) entfernt wird und worin die Menge des in Stufe (c) umgesetzten Alkohols mit einer Hydroxylgruppe in dem Bereich von etwa 1 bis etwa 2 Mol pro Mol vereintem Acrolein liegt.

3. Verfahren nach Anspruch 1, worin die Isocyanursäure-Acrolein-Reaktion in Stufe (b) bei einer Temperatur im Bereich von etwa 80 bis etwa 100°C durchgeführt wird und worin etwa 2 Mol Alkohol mit einer Hydroxylgruppe pro Mol vereintem Acrolein in Stufe (c) umgesetzt werden.

4. Verfahren nach Anspruch 1, worin der mild acide Zustand in Stufe (a) durch Zugabe von etwa 0,1 bis etwa 1,0 Mol Aminsalz pro 100 Mol Isocyanursäure erriecht wird und worin die Säure der Stufe (c) einen pKa-Wert von weniger als 3 aufweist und im Bereich von etwa 0,05 bis etwa 1 Gew.-% des Acrolein-Isocyanursäure-Addukts vorhanden ist.

5. Verfahren nach Anspruch 4, worin das Aminsalz das Triethylaminformiat ist.

6. Verfahren nach Anspruch 4, worin der Säurekatalysator mit einer anorganischen Base oder einem Ionenaustauscherharz nach Beendigung der Stufe (c) neutralisiert wird.

7. Verfahren nach Anspruch 1, worin der Alkohol mit einer Hydroxylgruppe ein $C_1$- bis $C_8$-Alkohol mit einer Hydroxylgruppe ist.

8. Verfahren nach Anspruch 1, worin der Alkohol mit einer Hydroxylgruppe Methylalkohol ist.

9. Acetal eines Isocyanursäure-Acrolein-Addukts und eines primären oder sekundären Alkohols mit einer Hydroxylgruppe, das nach dem Verfahren gemäß Anspruch 1 zu erhalten ist.

10. Acetal nach Anspruch 9, worin der Alkohol mit einer Hydroxylgruppe ein $C_1$- bis $C_8$-Alkohol ist.

11. Acetal nach Anspruch 9, worin der Alkohol mit einer Hydroxylgruppe der Methylalkohol ist.

12. Acetal nach Anspruch 9, 10 oder 11, worin das Addukt 1 bis 3 Mol vereintes Acrolein pro Mol Isocyanursäure enthält.

13. Acetal nach Anspruch 10, 11 oder 12, worin das Addukt 2 bis 3 Mol vereintes Acrolein pro Mol Isocyanursäure enthält.

14. Acetal-Zusammensetzung, enthaltend ein [3,3 - Di - (hydrocarbyloxy)propyl]isocyanurat.

15. Acetal-Zusammensetzung, enthaltend Tris[3,3 - di - (hydrocarbyloxy)propyl]isocyanurat.

16. Acetal-Zusammensetzung nach Anspruch 14 oder 15, worin die Hydrocarbylgruppe 1 bis 8 Kohlenstoffatome enthält.

17. Acetal-Zusammensetzung nach Anspruch 14 oder 5, worin die Hydrocarbylgruppe eine Methylgruppe ist.

18. Zusammensetzung, enthaltend eine polyfunktionelle Acetal-reaktive Verbindung und ein Acetal eines Acrolein-Isocyanursäure-Addukts und einen primären oder sekundären Alkohol mit einer Hydroxylgruppe, die nach dem Verfahren gemäß Anspruch 1 zu erhalten ist.

19. Zusammensetzung nach Anspruch 18, worin das Addukt etwa 1 bis etwa 3 Mol Acrolein, die pro Mol Isocyanursäure zugegeben werden, enthält, und mit etwa 1 bis etwa 2 Mol Alkohol mit einer Hydroxylgruppe pro Mol vereintem Acrolein acetalisiert ist.

20. Zusammensetzung nach Anspruch 18, worin der Alkohol mit einer Hydroxylgruppe ein $C_1$- bis $C_8$-Alkohol ist.

21. Zusammensetzung nach Anspruch 18, worin der Alkohol mit einer Hydroxylgruppe der Methylalkohol ist.

22. Zusammensetzung nach Anspruch 18, 19, 20 oder 21, worin das Molverhältnis von Acetal zu plyfunktioneller Acetal-reaktiver Verbindung im Bereich von etwa 1:m/2 bis etwa n/2:1 liegt, worin m die Funktionalität des Acetals ist und n die Funktionalität der polyfunktionellen Acetal-reaktiven Verbindung bedeutet und m und n mindestens etwa 2 sind.

23. Zusammensetzung nach Anspruch 22, worin die funktionellen Gruppen der Acetal-reaktiven Verbindung unter Alkohol, Thiol, Oxiran und Amid ausgewählt sind.

24. Zusammensetzung nach Anspruch 22, worin die Aldehydreaktive Verbindung eine Polyhydroxy-Verbindung ist.

25. Reaktionsprodukt der Zusammensetzung nach Anspruch 22, 23 oder 24.

26. Gegenstand, enthaltend das Reaktionsprodukt der Zusammensetzung nach Anspruch 22, 23 oder 24.

**Revendications**

1. Procédé de préparation d'un acétal qui consiste:

a. à faire une bouillie d'acide isocyanurique dans un milieu solvant d'un pH dans l'intervalle d'environ 5 à environ 7, contenant au moins environ une mole d'acroléine par mole d'acide isocyanurique;

b. à faire ajouter l'acroléine à l'acide isocyanurique à une température dans l'intervalle d'environ 50 à environ 120°C; et

c. à faire réagir le produit d'addition avec un monoalcool primaire ou secondaire en présence d'un acide fort dans des conditions qui provoquent le dégagement de l'eau libérée du milieu réactionnel.

2. Procédé selon la revendication 1, dans lequel la quantité d'acroléine au stade (a) est dans l'intervalle d'environ 1 à environ 6 moles par moles d'acide isocyanurique, dans lequel l'excès d'acroléine est éliminé à la fin du stade (b) et dans lequel la quantité de monoalcool ayant réagi au stade (c) est dans l'intervalle d'environ 1 à environ 2 moles par mole d'acroléine combinée.

3. Procédé selon la revendication 1, dans lequel la réaction acide isocyanurique-acroléine au stade (b) est effectuée à une température dans l'intervalle d'environ 80 à environ 100°C, et dans lequel environ 2 moles de monoalcool par mole d'acroléine combinée sont mises à réagir au stade (c).

4. Procédé selon la revendication 1, dans lequel le milieu faiblement acide au stade (a) est obtenu par addition d'environ 0,1 à environ 1,0 mole d'un sel d'amine pour 100 moles d'acide isocyanurique, et dans lequel l'acide du stade (c) a un $pk_a$ inférieur à 3 et est présent dans l'intervalle d'environ 0,05 à environ 1% en poids du produit d'addition acroléine-acide isocyanurique.

5. Procédé selon la revendication 4, dans lequel le sel d'amine est le formiate de triéthylamine.

6. Procédé selon la revendication 4, dans lequel le catalyseur acide est neutralisé avec une base minérale ou une résine échangeuse d'ions lorsque le stade (c) est terminé.

7. Procédé selon la revendication 1, dans lequel le monoalcool est une monoalcool en $C_1$ à $C_8$.

8. Procédé selon la revendication 1, dans lequel le monoalcool est l'alcool méthylique.

9. Acétal d'un produit d'addition acide isocyanurique-acroléine et d'un monoalcool primaire ou secondaire pouvant être obtenu par le procédé de la revendication 1.

10. Acétal selon la revendication 9, dans lequel le monoalcool est une alcool en $C_1$ à $C_8$.

11. Acétal selon la revendication 9, dans lequel le monoalcool est l'alcool méthylique.

12. Acétal selon les revendications 9, 10 ou 11 dans lequel le produit d'addition comprend de 1 à 3 moles d'acroléine combinée par mole d'acide isocyanurique.

13. Acétal selon les revendications 10, 11 ou 12, dans lequel le produit d'addition comprend de 2 à 3 moles d'acroléine combinée par mole d'acide isocyanurique.

14. Composition d'acétal comprenant un isocyanurate de [3,3 - di - (hydrocarbyloxy)propyle].

15. Composition d'acétal comprenant un isocyanurate de tris [3,3 - di - (hydrocarbyloxy)propyle].

16. Composition d'acétal selon les revendications 14 ou 15, dans laquelle le groupe hydrocarbyle contient 1 à 8 atomes de carbone.

17. Composition d'acétal selon les revendications 14 ou 15, dans laquelle le groupe hydrocarbyle est un groupe méthyle.

18. Composition comprenant un composé polyfonctionnel réagissant avec les acétals et un acétal d'un produit d'addition acroléine-acide isocyanurique et un monoalcool primaire ou secondaire pouvant être obtenu par le procédé de la revendication 1.

19. Composition selon la revendication 18, dans laquelle le produit d'addition comprend d'environ 1 à environ 3 moles d'acroléine ajoutée par mole d'acide isocyanurique et est acétalisé avec d'environ 1 à environ 2 moles de monoalcool par mole d'acroléine combinée.

20. Composition selon la revendication 18, dans laquelle le monoalcool est un alcool en $C_1$ à $C_8$.

21. Composition selon la revendication 18, dans laquelle le monoalcool est l'alcool méthylique.

22. Composition selon les revendications 18, 19, 20, ou 21, dans laquelle le rapport molaire de l'acétal au composé polyfonctionnel réagissant avec les acétals est dans l'intervalle d'environ 1:m/2 à environ n/2:1, où m est le fonctionnalité de l'acétal et n est la fonctionnalité du composé polyfonctionnel réagissant avec les acétals et m et n sont au moins environ 2.

23. Composition selon la revendication 22, dans laquelle les groupes fonctionnels du composé réactif avec les acétals sont choisis dans le groupe constitué des alcools, des thiols, des oxiranes et des amides.

24. Composition selon la revendication 22, dans laquelle le composé réagissant avec les aldéhydes est un composé polyhydroxylé.

25. Produit de réaction de la composition des revendications 22, 23 ou 24.

26. Article comprenant le produit de réaction de la composition des revendications 22, 23 ou 24.